(19) Europäisches Patentamt
European Patent Office
Office européen des brevets

(11) **EP 3 930 664 B1**

(12) **EUROPEAN PATENT SPECIFICATION**

(45) Date of publication and mention
of the grant of the patent:
**13.12.2023 Bulletin 2023/50**

(21) Application number: **20762771.2**

(22) Date of filing: **26.02.2020**

(51) International Patent Classification (IPC):
*A61K 8/02* (2006.01)     *A47K 10/16* (2006.01)
*A47L 13/17* (2006.01)     *D21H 11/18* (2006.01)
*D21H 27/00* (2006.01)     *D04H 1/425* (2012.01)
*D04H 1/4258* (2012.01)    *D04H 1/492* (2012.01)
*D04H 1/587* (2012.01)     *D04H 1/64* (2012.01)

(52) Cooperative Patent Classification (CPC):
**A47K 10/16; A47L 13/17; D04H 1/425;
D04H 1/4258; D04H 1/492; D04H 1/587;
D04H 1/64; D21H 11/18; D21H 27/002;**
A47K 2010/3266

(86) International application number:
**PCT/IL2020/050217**

(87) International publication number:
**WO 2020/174471 (03.09.2020 Gazette 2020/36)**

(54) **DISPERSIBLE WIPES REINFORCED WITH A BINDING AGENT**

MIT EINEM BINDEMITTEL VERSTÄRKTE DISPERGIERBARE TÜCHER

LINGETTES DISPERSIBLES RENFORCÉES PAR UN AGENT LIANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.02.2019 US 201962811623 P
26.11.2019 US 201916695317**

(43) Date of publication of application:
**05.01.2022 Bulletin 2022/01**

(73) Proprietor: **Wipeflush Ltd.
7528906 Rishon LeZion (IL)**

(72) Inventors:
• **JACKSON, David Martin
Alpharetta, Georgia 30022 (US)**

• **LUETTGEN, Chris
Roswell, Georgia 30076 (US)**
• **TOPROVSKY, Moshe
7528906 Rishon LeZion (IL)**

(74) Representative: **Pearl Cohen Zedek Latzer Baratz
UK LLP
The Gridiron Building
One Pancras Square
London N1C 4AG (GB)**

(56) References cited:
EP-A1- 2 781 652        WO-A1-2014/091413
WO-A1-2015/156713       WO-A2-2012/138552
JP-B2- 4 124 938        US-A1- 2003 008 591
US-A1- 2011 293 931     US-A1- 2014 259 484

EP 3 930 664 B1

**Description**

**FIELD OF THE INVENTION**

The present invention generally relates to dispersible wipes. More particularly, the present invention relates to dispersible wipes reinforced with a binding agent.

**BACKGROUND OF THE INVENTION**

Dispersible wipes or flushable wipes are wet wipes that may be thrown away into the sewage system to be dispersed (i.e., broken down) after being submerged in a large amount of water. Commonly used flushable wipes are made from cellulose pulp fibers similar to those used in conventional toilet paper. However, because cellulose pulp fibers tend to disintegrate and disperse very easily in watery environments, they are too weak (e.g., have low tensile strength) when wetted with a conventional wet wipe lotion, such that they cannot be pulled out from a package. Alternatively, if strongly bonded using conventional means, the commercial flushable wipes are too strong to disperse properly in the sewage system, thus may block a domestic sewage system such that they are practically non- flushable. Accordingly, there is a need for wet flushable/dispersible wipes that have the required strength to be pulled out from a package when wet with a wipe lotion and are fully dispersible in the presence of excess water, as in the sewage system.

WO 2014/091413 describes a wet-laid sheet material comprising MFC, suitable for producing hygiene tissues such as wet wipes.

EP 2 781 652 A1 discloses wet wipes comprising nanofibrillar cellulose.

**SUMMARY OF THE INVENTION**

The present invention relates to a wet wipe, more specifically to a dispersible wet wipe, as described in claim 1. The present invention also relates to the use of a base sheet for producing a wet wipe comprising a wetting lotion, as described in claim 2. Preferred embodiments of the invention are described in claims 1-8. wipe. The wet wipe includes a base sheet comprising short length fibers; a dispersible binding agent reinforcing the base sheet; and a wetting lotion. The dispersible binding agent is configured to bind the fibers of the base sheet when the dispersible wipe includes liquid at an amount of at most 400 wt% from the weight of the base sheet and to disperse in excess water if the amount of the water exceeds 500 wt%.

The dispersible binding agent is a non-adhesive agent selected from: microfibrillar cellulose fibers, nano crystalline cellulose fibrils, microfibrillated cellulose, nanofibrillated cellulose, or nanocellulose crystals.

In some embodiments, the dispersible binding agent is an adhesive emulsion. In some embodiments, the adhesive emulsion is selected from: Vinyl Acetate based emulsion, Acrylic terpolymer emulsions and the like.

The dispersible binding agent is present in an amount of 0.5-5 gram/square meter (gsm). In some embodiments, the dispersible binding agent is present in an amount of 1-2 gsm.

The base sheet is a nonwoven sheet made from short length fibers. In some embodiments, the reinforced base sheet is comprised of fibers of a denier of no more than 5.0. In some embodiments, the short length fibers include at least one of: bi-lobal (ribbon cross section) viscose, lyocell rayon polylactic acid (PLA) polymer fibers, cotton fibers, wood pulp, and the like. In some embodiments, the base sheet may have a weight of 30 - 100 gsm. In some embodiments, the base sheet may have a weight of 50 - 70 gsm. The short length fibers have maximum length of at most 15 mm. In some embodiments, the diameters of the short length fibers are between 15 and 80 microns. In some embodiments, the wetting lotion may include: water, alcohol, acetone, perfume, reagent, soap, emulsifier, pigment, rheology modifier, aromatic oil, friction reduction agent and any combination thereof.

In some embodiments, the wet wiper may have a tensile strength of between 38.61 N/ m and 96.53 N/m (100 and 250 gm force / inch) when the amount of wetting lotion added is 300 wt% from the weight of the base sheet.

**BRIEF DESCRIPTION OF THE DRAWINGS**

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings in which:

Fig. 1 shows an illustration of a wipe dispenser according to some embodiments of the invention;
Fig. 2 is an illusration of a structure of a wipe according to some embodiments of the invention;
Figs. 3A and 3B are images taken during a flushability test conducted according to some embodiments of the invention;

Fig. 4 is a flowchart of a method of making a wet wipe according to some embodiments of the invention; and

Fig. 5 is a flowchart of a method of making a wet wipe according to other embodiments of the invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

## DETAILED DESCRIPTION OF EMBODIMENTS OF THE INVENTION

In the following detailed description, numerous specific details are set forth in order to provide a thorough understanding of the invention. However, it will be understood by those skilled in the art that the present invention may be practiced without these specific details. In other instances, well-known methods, procedures, and components, modules, units and/or circuits have not been described in detail so as not to obscure the invention. Some features or elements described with respect to one embodiment may be combined with features or elements described with respect to other embodiments. For the sake of clarity, discussion of same or similar features or elements may not be repeated.

Some aspects of the invention may be related to wet wipes that are fully dispersible in sewage water but still strong enough to be pulled out from a package in an everyday use. Packages of wet wipes according to embodiments of the invention may have shelf life longer than 1 year (e.g., 1.5 years, 2 years and more), during which the packed wet wipes may not lose their mechanical strength. Such wipes when used and thrown into the sewage system may be fully dispersed in typically less than one hour.

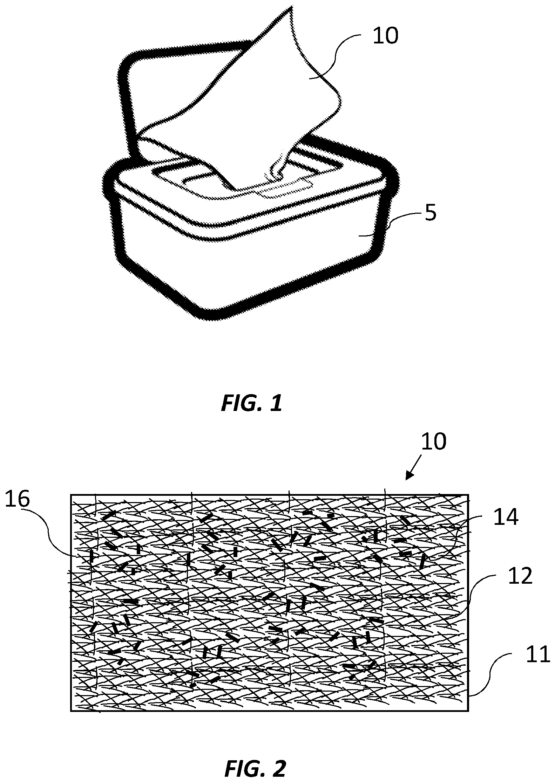

Reference is made to Fig. 1, which is an illusration of a wipe dispenser 5 that may include a plurality (e.g., 100) of wipes 10 according to some embodiments of the invention. In some embodiments, wipe dispenser 5 may have shelf life longer than 1 year (e.g., 1.5 years, 2 years and more), during which packed wet wipes 10 may not lose their mechanical strength. In some embodiments, wipe dispenser 5 may allow the dispensing/pulling out of a single wipe 10, each time, using any known method or configuration. For example, wipe dispenser 5 may be a single sheet wipe dispenser, roll control center-feed wipe dispenser, multifold wipes dispenser, and the like.

The structure of a wipe 10 according to some embodiments of the invention is better understood from the illusration in Fig. 2. Wipe 10 includes a base sheet 11 including short length fibers 12 and a dispersible binding agent 14 reinforcing base sheet 11. The wipe 10 is wet with a wetting lotion 16. The dispersible binding agent 14 is configured to bind short length fibers 12 of base sheet 11 when dispersible wipe 10 contains liquid at an amount of at most 400 wt.% from the weight of base sheet 11 and to disperse in excess water if the amount of the water exceeds 500 wt.% from the weight of base sheet 11.

In some embodiments, base sheet 11 may be a nonwoven sheet made from short length fibers 12 (e.g., dispersible fibers). As used herein, short length fibers are defined as fibers having a length of no more than 15mm. Therefore, short length fibers 12 may include synthetic and/or natural polymeric fibers, having a length of no more than 15mm, for example, less than 14 mm, less than 13mm, less than 12mm or less, in cut length. In some embodiments, the length of short length fibers 12 may at least 4 mm, for example, at least 5 mm, 6 mm or more. In embodiments, short length fibers may be in a range from 4 mm to 15 mm. In some embodiments, fibers having a length of no more than 15mm may allow good dispersibility of the fibers in sewage water. In some embodiments, fibers 12 may be capable of being wet or dry and formed into nonwoven sheet 11. In some embodiments, short length fibers may include dispersible fibers. As used herein, dispersible fibers may include any fiber materials which are dispersible, biodegradable and/or generally recognized as safe (GRAS). In some embodiments, short length fibers may include raw materials that may be composted or will degrade in the sewage active sludge process. For example, short length fibers 12 may be, at least one of: bi-lobal (ribbon cross section) viscose and/or lyocell rayon polylactic acid (PLA) polymer fibers (e.g., of less than 12mm in length), cotton fibers (e.g., of less than 3mm length), wood pulp (e.g., of less than 2.5mm length) and the like. In some embodiments, base sheet 11 may include more than one type of fiber, for example, cotton fibers and wood pulp. In some embodiments, the denier of fibers may be no more than 5.0, for example, 2.0. In some embodiments, the total weight of base sheet 10 may be 30-100 gram/square meter (gsm), for example, between 50-75 gsm.

In some embodiments, the diameters of fibers 12 may be between 15 and 80 microns, for example, between 25 and 50 microns.

In some embodiments, dispersible binding agent 14 may include any material capable of increasing the adhesion between short length fibers 12 when base sheet 11 is wet with wetting lotion 16. In some embodiments, dispersible binding agent 14 may be configured to disperse and/or dissolve in water when wipe 10 is flushed into the sewage system. In some embodiments, dispersible binding agent 14 may include any binding material which is dispersible, biodegradable and/or generally recognized as safe (GRAS). The dispersible binding agent 14 is added at an amount of 0.5-5 gsm, for example, 0.5-10 wt.% from the total dry weight of base sheet 11 reinforced with binding agent 14. In some embodiments, dispersible binding agent 14 may be added at an amount of 1-2 gsm, for example, 1-4 wt.% from the total dry weight of base sheet 11 reinforced with binding agent 14.

The dispersible binding agent 14 is a non-adhesive agent selected from: microfibrillar cellulose fibers, nano crystalline cellulose fibrils, microfibrillated cellulose, nanofibrillated cellulose, or nanocellulose crystals.

In some embodiments, binding agent 14 may include an adhesive emulsion, for example, Vinyl acetate based aqueous emulsion, Acrylic terpolymer emulsions and the like. In some embodiments, the total dry weight of the reinforced base sheet may be 35-105 gsm, for example, 50-75 gsm.

In some embodiments, wetting lotion 16 may be any lotion known in the art, for example, a lotion composition for a skin cleaning wipe may include: water, a wetting agent /soap and a rheology modifier and/or a friction reducing agent, to create a gliding feeling, complementary emollients, emulsifiers, and preservatives are also added. In some embodiments, the wetting lotion may include: water, alcohol, acetone, perfume, reagent, soap, emulsifier, pigment, rheology modifier, aromatic oil, friction reduction agent and any combination thereof. In some embodiments, dispersible wet wipe 10 may contain wetting lotion at an amount of at most 400 wt.% from the weight of base sheet 11, for example, at an amount of 300 wt.% from the weight of base sheet 11.

In some embodiments, the tensile strength of wet wipe 10 may between 100 to 250 gm force/inch when the amount of wetting lotion added is 300 wt% from the weight of the base sheet. The tensile test measurements were conducted using Instron Model 1122. Sample test strips were cut to dimensions of 2 x 6 inches (1 inch = 2.54 cm) with the length perpendicular to the non-woven elongation lines. Each test piece was weighed prior to tensile testing. Tensile testing was made with an elongation speed of 12 inches per minute, jaw separation 4 inches. 6 replicate measurements were made per sample. 6 samples are weighed then placed in a tray containing 300 wt.% their weight in a water solution with 1% dishwashing soap. Petri dish covers with 100 gsm weights were placed on top of the wetted stack of samples and left standing for 5 minutes. Tensile testing proceeded as above with the sample test pieces weighed post soaking to check how much water was absorbed.

When dispersible wipe 10 is further wet with water, for example, while flushed into the sewage system, the amount of water dissolved by wipe 10 may exceed 500 wt.% from the weight of base sheet 11. In such conditions, binding agent 14 is dissolved/dispersed in the excess water, thus allowing dispersible fibers 12 of base sheet 11 to be fully dispersed in the excess water. 60 wt.% of wet wipe 10 is dispersed in excess water already after 30 minutes.

Flushability testing was conducted according to Guidelines for Assessing Flushability of Disposable Nonwoven Products, INDA and EDANA (May 2018). About 0.6 grams of wipes according to embodiments of the invention were cut as large rectangular pieces and were added to 125 milliliters of water in a flask. The wipes were agitated with a lab wrist action shaker for 2 minutes. The flask contents were then poured into a 5/16th mesh (8 mm) screen and the retained contents dried at 105 °C and equilibrated to 50% RH (relative humidity) and weight.

The Flushability was calculated using equation 1:

$$\% \text{ Flushability} = [1-(\text{Original sample weight} - \text{flushed sample weight})/\text{original sample weight}] \times 100.$$

The samples were made from a base sheet that includes about 20 wt.% bi-lobal (ribbon cross section) viscose, 50 weight% cotton fibers and 30 wt.% wood pulp reinforced with microfibrillar cellulose fibers. The flushability of the samples were varied from 0-30%.

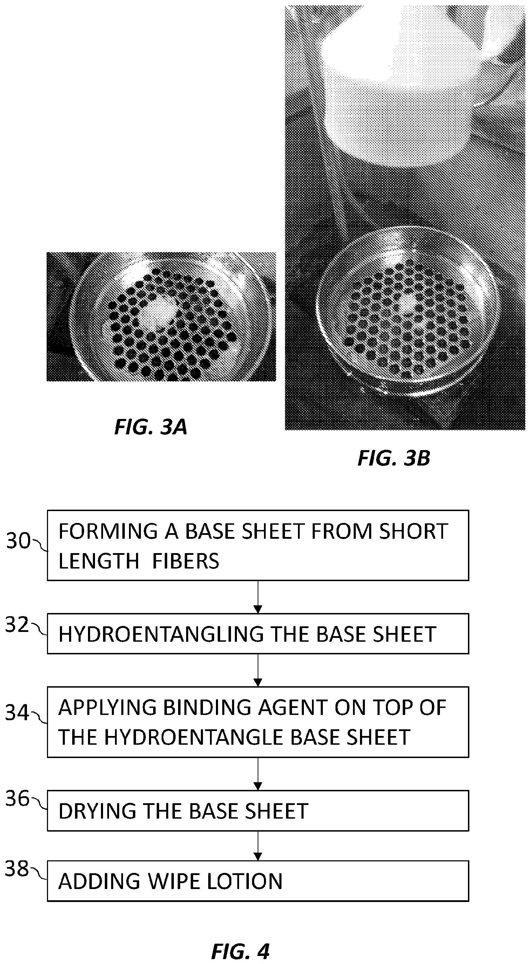

Reference is now made to Figs. 3A and 3B which shows images taken during a flushability test according to some embodiments of the invention. Fig. 3A shows the wipe sample at the beginning of the flushability test and Fig. 3B what left from the wipe at the end of the flushability test. As would have been clear to one skilled in the art, a longer flushability test will result in complete disintegration of the test wipe.

Reference is now made to Fig. 4 which shows a flowchart of a method of making a wet wipe according to some embodiments of the invention. In box 30, a base sheet may be formed using the short length fibers. For example, short length fibers 12 may be processed using any known paper machine. In box 32, base sheet may be hydroentangled using, for example, low energy hydroentanglement and standard HE (hydroentangled) water needling technique. The energy used may be less than 500 PSI, for example, 250 PSI (1 PSI = 6.89476 kPa).

In box 34, a binding agent may be applied on top of the hydroentangled base sheet. For example, binding agent 14 may be sprayed, printed, etc. on top of hydroentangled base sheet 11 in order to reinforce the adhesion between fibers 12 of base sheet 11. In box 36, the reinforce base sheet may be dried using any known method, for example, by air impingement or convection heating methods. In box 38, at most 400 wt.% of a wetting lotion may be added to the reinforced base sheet. For example, 300 wt.% (e.g., 3 times the weight of the dried reinforced base sheet) of lotion may be soaked into the reinforced base sheet, to form the wet wipe.

In some embodiments, the wet wipe may further be packed in a packed inside a wipe dispenser, for example, wipe dispenser 5.

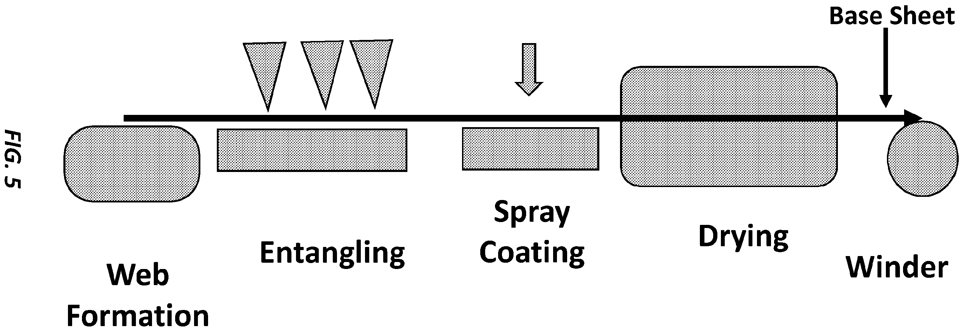

Reference is now made to Fig. 5 which shows an aspect of the method of making a wet wipe according to some embodiments of the invention. In order to increase the wet strength of the wiper sheet, MicroFibrillar Cellulose (MFC) is added to the surface of the sheet to increase hydrogen bonding. The MFC is typically added at approximately 3% by weight to the sheet. Usually less than 10%. For a standard weight wipe of 65gsm this is approximately 2gsm of coat weight.

In accordance with some embodiments of the present invention, the MFC may be sprayed on one or both sides of the sheet, or indeed coated by other means such as slot coating / Meyer rod etc. The coating may be added following the sheet formation and entangling process as shown in Fig. 5. The MFC is typically coated from a dispersion of the MFC in water at 1 - 2% solids.

While certain features of the invention have been illustrated and described herein, many modifications, substitutions, changes, and equivalents may occur to those skilled in the art. It is, therefore, to be understood that the appended claims are intended to cover all such modifications and changes within their scope.

Various embodiments have been presented. Each of these embodiments may of course include features from other embodiments presented, and embodiments not specifically described may include various features described herein.

## Claims

1. A wet wipe comprising:

   a base sheet comprising short length fibers,

   wherein the short length fibers have maximum length of at most 15 mm;
   wherein the surface of the base sheet further comprises a MicroFibrillar Cellulose "MFC" layer,

   wherein the MFC layer is approximately 3% by weight of the base sheet and sprayed on one or both sides of the base sheet, or coated by other means; and
   wherein the MFC layer is added following the sheet formation and entangling process;

   a dispersible binding agent reinforcing the base sheet,

   wherein the dispersible binding agent is a non-adhesive agent selected from microfibrillar cellulose fibers, nano crystalline cellulose fibrils, microfibrillated cellulose, nanofibrillated cellulose, or nanocellulose crystals;
   wherein the dispersible binding agent is in an amount of 0.5-5 gram/square meter (gsm); and
   wherein the dispersible binding agent is configured to bind the fibers of the base sheet when the wipe comprises liquid at an amount of at most 400wt% from the weight of the base sheet and for 60 wt% of the wipe to be dispersed in excess water already after 30 minutes if the amount of the water exceeds 500 wt%, as determined according to the flushability test described herein;

   and a wetting lotion.

2. Use of a base sheet comprising short length fibers and a dispersible binding agent reinforcing the base sheet for producing a wet wipe comprising a wetting lotion,

   wherein the short length fibers have maximum length of at most 15 mm;
   wherein the surface of the base sheet further comprises a MicroFibrillar Cellulose "MFC" layer,

   wherein the MFC layer is approximately 3% by weight of the base sheet and sprayed on one or both sides of the base sheet, or coated by other means; and
   wherein the MFC layer is added following the sheet formation and entangling process;

   wherein the dispersible binding agent is a non-adhesive agent selected from microfibrillar cellulose fibers, nano crystalline cellulose fibrils, microfibrillated cellulose, nanofibrillated cellulose, or nanocellulose crystals;
   wherein the dispersible binding agent is in an amount of 0.5-5 gram/square meter (gsm); and
   wherein the dispersible binding agent is configured to bind the fibers of the base sheet when the wipe comprises liquid at an amount of at most 400wt% from the weight of the base sheet and for 60 wt% of the wipe to be dispersed in excess water already after 30 minutes if the amount of the water exceeds 500 wt%, as determined according to the flushability test described herein.

3. The wet wipe of claim 1 or the use of the base sheet of claim 2, wherein the dispersible binding agent is in an amount of 1-2 gsm.

4. The wet wipe of claim 1 or the use of the base sheet of claim 2, wherein the reinforced base sheet is comprised of fibers of a denier of no more than 5.0.

5. The wet wipe of claim 1 or the use of the base sheet of claim 2, wherein the short length fibers comprise at least one of: bi-lobal (ribbon cross section) viscose, lyocell rayon polylactic acid (PLA) polymer fibers, cotton fibers and wood pulp.

6. The wet wipe of claim 1 or the use of the base sheet of claim 2, wherein the base sheet has a weight of 30 - 100 gsm.

7. The wet wipe of claim 1 or the use of the base sheet of claim 2, wherein the diameters of the short length fibers are between 15 and 80 microns.

8. The wet wipe of claim 1 or the use of the base sheet of claim 2, wherein the wet wipe has a tensile strength of between 38.61 N/m and 96.53 N/m (100 and 250 gm force / inch) when the amount of wetting lotion added is 300 wt% from the weight of the base sheet.


**Patentansprüche**

1. Feuchttuch, wie folgt umfassend:

ein Grundblatt, das Fasern kurzer Länge umfasst,

wobei die Fasern kurzer Länge eine Maximallänge von höchstens 15 mm haben;
wobei die Oberfläche des Grundblatts ferner eine Schicht MicroFibrillar-Zellulose ("MFC"-Schicht) umfasst,

wobei die MFC-Schicht etwa 3 Gew.-% des Grundblatts ist und auf eine Seite oder auf beide Seiten des Grundblatts aufgesprüht oder mit anderen Mitteln aufgebracht wird; und
wobei die MFC-Schicht im Anschluss an die Blattausbildung und den Verflechtungsprozesses hinzugefügt wird;

dispergierbares Bindemittel, das das Grundblatt verstärkt,

wobei das dispergierbare Bindemittel ein nicht-adhäsives Mittel ist, das unter mikrofibrillären Zellulosefasern, Nanokristall-Zellulosefibrillen, mikrofibrillierter Zellulose, nanofibrillierter Zellulose oder Nanozellulose-Kristallen ausgewählt ist;
wobei das dispergierbare Bindemittel in einer Menge von 0,5-5 g/Quadratmeter (g/m$^2$) vorliegt; und
wobei das dispergierbare Bindemittel dazu konfiguriert ist, die Fasern des Grundblatts zu binden, wenn das Feuchttuch eine Feuchtigkeitsmenge von höchstens 400 Gew.-% in Bezug auf das Gewicht des Grundblatts umfasst, und wobei 60 Gew.-% des Tuchs schon nach 30 Minuten in einem Wasserüberschuss dispergiert werden, wenn die Wassermenge 500 Gew.-% überschreitet, wie gemäß dem vorliegend beschriebenen Abspülbarkeitstest bestimmt wird;

und eine Benetzungslotion.

2. Verwendung eines Grundblatts, umfassend Fasern kurzer Länge und ein dispergierbares Bindemittel, das das Grundblatt verstärkt, zum Produzieren eines Feuchttuchs, das eine Benetzungslotion umfasst,

wobei die Fasern kurzen Länge eine maximale Länge von höchstens 15 mm haben;
wobei die Oberfläche des Grundblatts ferner eine Schicht MicroFibrillar-Zellulose ("MFC"-Schicht) umfasst,

wobei die MFC-Schicht etwa 3 Gew.-% des Grundblatts ist und auf eine Seite oder auf beide Seiten des Grundblatts aufgesprüht oder mit anderen Mitteln aufgebracht wird; und

wobei die MFC-Schicht im Anschluss an die Blattausbildung und den Verflechtungsprozesses hinzugefügt wird;

wobei das dispergierbare Bindemittel ein nicht-adhäsives Mittel ist, das unter mikrofibrillären Zellulosefasern, Nanokristall-Zellulosefibrillen, mikrofibrillierter Zellulose, nanofibrillierter Zellulose oder Nanozellulose-Kristallen ausgewählt ist;

wobei das dispergierbare Bindemittel in einer Menge von 0,5-5 g/Quadratmeter (g/m$^2$) vorliegt; und wobei das dispergierbare Bindemittel dazu konfiguriert ist, die Fasern des Grundblatts zu binden, wenn das Feuchttuch eine Feuchtigkeitsmenge von höchstens 400 Gew.-% in Bezug auf das Gewicht des Grundblatts umfasst, und wobei 60 Gew.-% des Tuchs schon nach 30 Minuten in einem Wasserüberschuss dispergiert werden, wenn die Wassermenge 500 Gew.-% überschreitet, wie gemäß dem vorliegend beschriebenen Abspülbarkeitstest bestimmt wird.

3. Feuchttuch nach Anspruch 1 oder Verwendung des Grundblattes nach Anspruch 2, wobei das dispergierbare Bindemittel in einer Menge von 1-2 g/ m2 vorliegt.

4. Feuchttuch nach Anspruch 1 oder Verwendung des Grundblattes nach Anspruch 2, wobei das verstärkte Grundblatt Denierfasern von nicht mehr als 5,0 umfasst.

5. Feuchttuch nach Anspruch 1 oder Verwendung des Grundblattes nach Anspruch 2, wobei die Fasern kurzer Länge mindestens eines hiervon umfassen: bilobale (Ribbon-Querschnitt) Viskose, Lyocell-Rayon-Polymilchsäure (PLS)-Polymerfasern, Baumwollfasern und Holzpülpe.

6. Feuchttuch nach Anspruch 1 oder Verwendung des Grundblattes nach Anspruch 2, wobei das Grundblatt ein Gewicht von 30-100 g/m2 hat.

7. Feuchttuch nach Anspruch 1 oder Verwendung des Grundblattes nach Anspruch 2, wobei der Durchmesser der Fasern kurzer Länge jeweils zwischen 15 und 80 Mikron ist.

8. Feuchttuch nach Anspruch 1 oder Verwendung des Grundblattes nach Anspruch 2, wobei das Feuchttuch eine Zugfestigkeit zwischen 38,61 N/m und 96,53 N/m (100 und 250 gm Kraft / Zoll) hat, wenn die Menge der zugesetzten Benetzungslotion 300 Gew.-% in Bezug auf das Gewicht des Grundblatts ist.

## Revendications

1. Lingette humide comprenant :

une feuille de base comprenant des fibres courtes,

les fibres courtes ayant une longueur maximale de 15 mm ;
la surface de la feuille de base comprenant en outre une couche de cellulose microfibrillaire « MFC »,

la couche MFC représentant environ 3 % en poids de la feuille de base et pulvérisée sur l'une ou sur les deux faces de la feuille de base, ou enduite par d'autres moyens ; et
la couche MFC étant ajoutée après le processus de formation et d'emmêlement de la feuille ;

un liant dispersable renforçant la feuille de base,

le liant dispersable étant un agent non adhésif choisi parmi les fibres de cellulose microfibrillaires, les fibrilles de cellulose nanocristalline, la cellulose microfibrillée, la cellulose nanofibrillée ou les cristaux de nanocellulose ;
le liant dispersable étant présent en une quantité de 0,5 à 5 grammes/mètre carré (g/m$^2$) ; et
le liant dispersable étant configuré pour lier les fibres de la feuille de base lorsque la lingette comprend un liquide en une quantité maximale de 400 % en poids par rapport au poids de la feuille de base et pour que 60 % en poids de la lingette soit déjà dispersée dans l'excès d'eau après 30 minutes si la quantité d'eau dépasse 500 % en poids, comme déterminé selon le test de possibilité d'élimination dans les toilettes décrit ici ;

et une lotion mouillante.

2. Utilisation d'une feuille de base comprenant des fibres courtes et un liant dispersable renforçant la feuille de base pour produire une lingette humide comprenant une lotion mouillante,

les fibres courtes ayant une longueur maximale de 15 mm ;
la surface de la feuille de base comprenant en outre une couche de cellulose microfibrillaire « MFC »,

la couche MFC représentant environ 3 % en poids de la feuille de base et étant pulvérisée sur l'une ou sur les deux faces de la feuille de base, ou enduite par d'autres moyens ; et
la couche MFC étant ajoutée après le processus de formation et d'emmêlement de la feuille ;

le liant dispersable étant un agent non adhésif choisi parmi les fibres de cellulose microfibrillaires, les fibrilles de cellulose nanocristalline, la cellulose microfibrillée, la cellulose nanofibrillée ou les cristaux de nanocellulose ;
le liant dispersable étant présent en une quantité de 0,5 à 5 grammes/mètre carré (g/m$^2$) ; et
le liant dispersable étant configuré pour lier les fibres de la feuille de base lorsque la lingette comprend un liquide en une quantité maximale de 400 % en poids par rapport au poids de la feuille de base et pour que 60 % en poids de la lingette soit déjà dispersée dans l'excès d'eau après 30 minutes si la quantité d'eau dépasse 500 % en poids, comme déterminé selon le test de possibilité d'élimination dans les toilettes décrit ici ;

3. Lingette humide selon la revendication 1 ou utilisation de la feuille de base selon la revendication 2, dans laquelle le liant dispersable est présent en une quantité de 1 à 2 g/m2.

4. Lingette humide selon la revendication 1 ou utilisation de la feuille de base selon la revendication 2, dans laquelle la feuille de base renforcée est constituée de fibres d'un denier maximum de 5,0.

5. Lingette humide selon la revendication 1 ou utilisation de la feuille de base selon la revendication 2, dans laquelle les fibres courtes comprennent au moins l'un parmi : de la viscose bilobée (section transversale du ruban), du lyocell, de la rayonne, des fibres polymères d'acide polylactique (PLA), des fibres de coton et de la pâte de bois.

6. Lingette humide selon la revendication 1 ou utilisation de la feuille de base selon la revendication 2, dans laquelle la feuille de base présente un grammage de 30 à 100 g/m$^2$.

7. Lingette humide selon la revendication 1 ou utilisation de la feuille de base selon la revendication 2, dans laquelle les diamètres des fibres courtes sont compris entre 15 et 80 microns.

8. Lingette humide selon la revendication 1 ou utilisation de la feuille de base selon la revendication 2, dans laquelle la lingette humide a une résistance à la traction comprise entre 38,61 N/m et 96,53 N/m (100 et 250 g force/pouce) lorsque la quantité de lotion mouillante ajoutée représente 300 % en poids du poids de la feuille de base.

**FIG. 1**

**FIG. 2**

**FIG. 3A**

**FIG. 3B**

30 — FORMING A BASE SHEET FROM SHORT LENGTH FIBERS

32 — HYDROENTANGLING THE BASE SHEET

34 — APPLYING BINDING AGENT ON TOP OF THE HYDROENTANGLE BASE SHEET

36 — DRYING THE BASE SHEET

38 — ADDING WIPE LOTION

**FIG. 4**

**FIG. 5**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014091413 A

- EP 2781652 A1